# EUROPEAN PATENT APPLICATION

(11) **EP 3 995 148 A1**
(43) Date of publication of application: **11.05.2022**
(21) Application number: 20206822.7
(22) Date of filing: 10.11.2020
(51) Int. Cl.: A61K 41/00, A61P 15/00

(54) **USE OF PHENOTHIAZINE PHOTOSENSITIZERS FOR TREATMENT OF SEXUALLY TRANSMISSIBLE INFECTIONS**

(71) Applicant: Arentz, Jochen, 22301 Hamburg (DE); Albrecht, Hans, 81479 München (DE)
(72) Inventor: Arentz, Jochen, 22301 Hamburg (DE); Albrecht, Hans, 81479 München (DE)
(74) Representative: Habermann, Hruschka & Schnabel

(57) **Abstract**

The present invention relates to the treatment and/or prevention of sexually transmissible infections, in particular sexually transmissible viral infections, using phenothiazine photosensitizers as well as to the treatment and/or prevention of diseases caused thereby, occurring in the urogenital tract or in the oropharynx of a subject, preferably a human subject. The invention furthermore relates to kits for use in the inventive treatments comprising a device of the invention and suitable light source.

## Description

The present invention relates to the treatment and/or prevention of sexually transmissible infections, in particular sexually transmissible viral infections, using phenothiazine photosensitizers as well as to the treatment and/or prevention of diseases caused thereby, occurring in the urogenital tract or in the oropharynx of a subject, preferably a human subject. The invention furthermore relates to kits for use in the inventive treatments comprising a device of the invention and suitable light source.

Photosensitizers have been used for photodynamic therapy (PDT) of infections by bacteria and fungi as well as cancer and tumours. Photosensitizers are generally dyes which are transferred to a higher energetic state by absorption of light typically in the visible wavelength range. In the photo-activated state the photosensitizer is able to transfer, as a donor, at least part of the energy to an acceptor molecule. For application in PDT, the energy transfer leads, at least in part, to reactive oxygen species (ROS) such as the superoxide radical which in turn damages various cellular components, especially the cell membrane, of the target cells such as tumour cells, bacteria, or fungal cells, by redox reactions. PDT is mostly applied through local application of the dye and light typically emitted by monochromatic and/or coherent light sources.

The technical problem underlying the present invention is to provide improved treatment of sexually transmissible viral infections as well as therapeutic devices and kits for use in such the treatments.

In particular, the present invention provides, under a first aspect, the use of at least one phenothiazine-derived photosensitizer in the treatment of sexually transmissible infections.

Sexually transmissible infections typically occur and persist, respectfully, in the urogenital tract, but also include infections of the oropharynx as well as parts of the skin related to the urogenital tract and/or the oropharynx. In order to provide the photosensitizer in the required amount and concentration at the region where the targeted infectious agent is present or at least presumed to be present, it is preferred that the photosensitizer is administered topically to the body region or regions, respectively, where the infectious agent is present or at least presumed to be present. In preferred embodiments the one or more photosensitizer(s) is/are applied to the urogenital tract or at least a part thereof such as preferably by intra-vaginal and/or intra-urethral and/or intra-uterine administration. In other embodiments, the one or more photosensitizer(s) is/are administered to the oropharynx or at least a part thereof, such as preferably by intra-oral and/or intra-pharyngeal administration. In other embodiments of the invention, the photosensitizer can also be applied to the skin of the subject to be treated, for example by administration to the facial skin, or parts thereof such as preferably lips, cheeks chin and/or eye surrounding skin etc., and/or the skin of the genital area, or at least part or parts thereof, including vulva, labia, penis, scrotum, perineum and/or perianal skin, as well as related parts of the skin, i.e. perigenital skin such as preferably the groins, thighs, nates and/or lower back in case the infecting agent infects or persists in, respectively, such body areas.

As mentioned above, the photosensitizer needs to be activated in order to exert its function of at least damaging or, optimally, eliminating the infecting agent from the subject to be treated, which is accomplished by light emission on the part or region of the body of the subject in need of the inventive treatment where the photosensitizer has been applied to. Typically, the light emission is directed to the urogenital tract, or at least, and preferably, a part thereof, of the subject, or the oropharynx, or skin, where applicable, of the subject. The light for irradiating at least a part of the desired part of the subject's body has a wavelength, or at least comprises a wavelength, and typically also an intensity, preferably in terms of energy density, i.e. applied energy per surface area, causing the photosensitizer to be transferred to an active state as outlined above.

In certain embodiments of the invention the treatment comprises irradiating at least a part of the urogenital tract of a subject from outside of the subject with light comprising or having a wavelength causing the photosensitizer to be transferred to an active state. It is conceived that one or more different parts of the urogenital tract may be irradiated one or more times for a time effective to provide the sufficient light energy so as to effect the inventive transfer of the photosensitizer, in particular the effective amount administered to the urogenital tract, or one or more parts thereof, to the active state at least in the irradiated part or region, or whole of, respectively, of the urogenital tract. Parts of the urogenital tract which may be conveniently irradiated from the outside of the subjects body include, e.g. vulva, vagina, cervix, uterus, penis, scrotum, bladder and/or ureter, especially the lower part thereof, in particular in the case of male subjects, the penis part of the ureter.

In other embodiments of the invention the treatment comprises irradiating at least a part of the oropharynx of a subject from outside of the subject with light comprising or having a wavelength causing the photosensitizer to be transferred to an active state. It is conceived that one or more different parts of the oropharynx may be irradiated one or more times for a time effective to provide the sufficient light energy so as to effect the inventive transfer of the photosensitizer, in particular the effective amount administered to the oropharynx, or one or more parts thereof, to the active state at least in the irradiated part or region, or whole, respectively, of the oropharynx. Parts of the oropharynx which may be conveniently irradiated from the outside of the subjects body include mouth (preferably, subject's mouth can be irradiated while in the opened or at least partially opened state), lips, cheeks (one side or both sides, preferably left and right cheek simultaneously or sequentially), neck, throat, chest and/or back of the subject to be treated. It is to be understood that the term "from the outside of the subject's body" means that the irradiation is directed to the skin of the subject in the respective area, in the following also denoted as "transcutaneous irradiation". In an alternative embodiment of an "irradiation from the outside of the subject's body", as outlined before, the mouth of the subject can be irradiated in an open state where the emitted light will irradiate the oropharynnx of the treated subject. In any case, however, irradiation from the outside of the subject's body means that the light-emitting device is not introduced into any parts of the body of the subject treated, in particular the urogenital tract or oropharynx, respectively.

In other preferred embodiments of the invention at least a part of the region to be treated, in particular the urogenital tract and/or the oropharynx, respectively, of the subject in the need of the inventive treatment is irradiated within the subject's body, preferably by using a light source which is introduced into the infected or at least suspected to be infected subject. In an embodiment thereof, the light source is equipped with fibre optical elements, e.g. a waveguide, dimensioned, in particular with respect to its diameter and length, to be introduced intra-vaginal and/or intra-urethral and/or intra-uterine and/or intra-orally and/or intra/pharyngeally and pushed to the desired site of light emittance or irradiation, respectively, corresponding to the site(s) of application of the photosensitizer as outlined above. The irradiation of the subject from inside the body, preferably via suitable fibre optics, can be directed to one or more desired parts of the urogenital tract such as preferably vagina, ureter, bladder or uterus. For female subjects, a particularly preferred mode of irradiation is intra-vaginally, and more preferably an intra-vaginal irradiation of the cervix, preferably in case of a diagnosed or suspected, respectively, HPV infection as further outlined herein below. In the case of male subject, a highly preferred mode of irradiation is within the lower part of the ureter, preferably the urethral part within the penis, for example preferably in case of a diagnosed or suspected infection with HPV as further outlined herein below. With respect to the irradiation of the oropharynx within the subject's body is preferably an intra-oral and/or pharyngeal irradiation. One preferred example of irradiation of the oropharynx, or part thereof after application of the one or more photosensitizer to the respective area, is in the case of HPV infection as further described herein below. Fibre optics suitable for use in the invention are generally known in the art and commercially available, for example from CeramOptec GmbH, Bonn, Germany, Karl Storz GmbH, Tuttlingen, Germany, and various other commercial suppliers.

The light source used for irradiation in the inventive treatments, which, according to the invention, may refer to a light emitting element of a corresponding device or the device *per se*, is preferably a device comprising a light emitting diode (LED). For convenience of use, the LED device is, particularly when for use from outside of the subject's body, a handheld light emitting device, more preferably a handheld LED device such as a device having the form and comprising the typical parts of, respectively, a torch or flashlight, respectively. In certain embodiments of the invention the light source emits coherent light, in which case the light source is a laser device emitting light of a specific wavelength. The laser device can be an LED laser device, and in certain embodiments, it may be a handheld laser LED device, as outlined above. In other preferred embodiments, the light source or light emitting device, respectively, can be a light source or device, respectively, emitting non-coherent light, preferably an LED device, more preferably a handheld LED device. It is a surprising finding according to the invention that it is sufficient for attaining substantial and strong virus inhibition to use a broadband light source, more preferably a broadband LED device, more preferably a broadband handheld LED device. In preferred embodiments of broadband LED devices, the LED light source is doted with Cer which shifts the potential dominant blue part (shorter wavelength) of the emitted light to longer wavelengths. In other preferred embodiments the LED devices can be one emitting non-coherent monochromatic light, preferably having a wavelength as further described below.

Phenothiazine-derived photosensitizers for use in the invention are photoactivatable phenothiazine derivatives, more preferably photoactivatable phenothiazine dyes. Phenothiazine photosensitizers for use in the invention typically have a structure according to the following general formula (I): 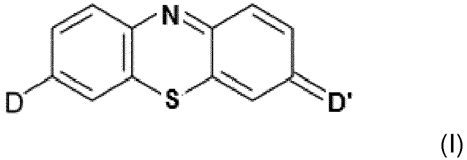 with D and D' each being an electron donor group, and wherein the positions C-1, C-2, C-4, C-6, C-8 and/or C-9 of the phenothiazine ring system may be substituted by a substituent preferably selected from substituted or unsubstituted lower alkyl, preferably C₁₋₃-alkyl, particularly preferred methyl, or lower alkenyl, preferably C₂₋₃-alkenyl, amino, halo, and cyano. Preferably, D and D' are each independently a NRR' group with R and R' being preferably independently selected from H, substituted or unsubstituted lower alkyl, preferably C₁₋₃-alkyl, particularly preferred methyl, and lower alkenyl, preferably C₂₋₃-alkenyl.

Particularly preferred phenothiazine derivatives for use in the invention are methylene blue, new methylene blue, toluidine blue and mixtures thereof.

The photosensitizer for use in the invention, preferably a phenothiazine derivative as described above, is typically applied in form of a composition containing at least one photosensitizer such as a photoactivatable phenothiazine in combination with at least one excipient and/or diluent, preferably at least one pharmaceutically acceptable excipient and/or diluent. Preferred diluents/excipients for use in the invention are typically water, preferably sterilized water, more preferably sterilized distilled or deionized water, or aqueous solutions, e.g. saline solutions or buffered saline solutions such as PBS, whereby it is understood that such diluents and/or excipients are preferably sterilized.

It has been surprisingly found according to the invention that photosensitizers as taught herein, preferably phenothiazine derivatives of above formula (I), particularly preferred methylene blue, new methylene blue, toluidine blue or mixtures thereof, can be applied in comparatively very low concentrations for effectively combating various sexually transmissible infections causing various conditions as described above. In preferred embodiments the composition comprises a photosensitizer for use in the invention, particularly preferred a phenothiazine derivative as defined herein, in a concentration of from about 0.00001 % (w/v) to about 0.1 % (w/v), preferably from about 0,0001 % (w/v) to about 0,001 % (w/v).

In the case of infection in the urogenital tract, the photosensitizer, preferably contained in a composition as outlined above, is preferably administered to the urogenital tract, or part thereof, of the subject through intra-vaginal and/or intra-urethral and/or intra-uterine administration. In certain embodiments, the photosensitizer is administered via intra-vaginal administration to the cervix of a female subject. For example, the photosensitizer, in particular the composition containing at least one photosensitizer (i.e. one or more of such photosensitizers) as outlined above may be administered via a catheter or tube or a similar device generally known in the art. For administration to parts of the urogenital tract, tube or catheter systems known in the art equipped with a syringe reservoir at its proximal part or end, respectively, can be used. Typical systems may contain also camera and corresponding light systems. In preferred embodiments for intra-nasal and/or intra-oral and/or intra-pharyngeal administration for application in the oropharynx, devices such as pipettes and syringes may be used. In preferred embodiments for application in the oropharynx, allowing for reaching the upper and lower parts of the oropharynx of the subject to be treated, the photosensitizer(s) is (are) preferably administered by use a device configured to administer the photosensitizer(s), preferably a composition containing the photosensitizer(s), in spray or nebulized from. Typically, such a device comprises a nebulizer element and a container comprising a composition containing one or more photosensitizers as described above.

A nebulizer device according to the invention, or for use in the inventive treatments, can be included in, or formed from, respectively, a kit of parts for intra-oral and intra-nasal application. For example, a kit can preferably comprise a container comprising a photosensitizer composition as described herein and one or more application adapters sized to apply the nebulized composition one or more parts of the respiratory tract, e.g. an adapter for intra-nasal application and an adapter for intra-oral application. In a variant of a medical kit according to the invention the kit comprises an empty nebulizer container and the photosensitizer composition in a separate container, wherein, before use, the photosensitizer composition is transferred into the nebulizer container.

The invention is also directed to the use of such pharmaceutical or medical, respectively, devices and/or kits as outlined above for the treatment of sexually transmissible infection as described herein by administration of the photosensitizers to the various parts of the subject in need of the inventive treatment.

As already noted above, the wavelength or wavelength spectrum of the light emitted by the light source depends on the particular photosensitizer. Preferred phenothiazine photosensitizers for use in the invention typically absorb light in the visible to near infra red light of the electromagnetic wave spectrum.. For example, in the visible spectrum, methylene blue absorbs light in the wavelength region of from about 530 nm to about 700 nm, new methylene absorbs light in the wavelength region of from about 500 nm to about 700 nm, and toluidine blue absorbs light in the region of from about 520 nm to about 700 nm. In preferred embodiments, the photosensitizer is typically activated by irradiation with light have a wavelength of wavelengths, respectively, in the range of from about 500 nm to about 820 nm. As already indicated above, the light source or light emitting device, respectively, for use in the invention emits light typically within this wavelength region, whereby the light source can emit light in broadband region, preferably, of the above indicated visible to near infrared spectrum, preferably of about 500 nm to about 820 nm. Particularly preferred, the wavelength used for activating the photosensitizer, preferably one or more of the above-described phenothiazines, preferably, methylene blue and/or new methylene blue and/or toluidine blue, most preferred methylene blue, is at or around 590 nm such as from about 550 nm to about 630 nm, preferably about 570 to about 610 nm, more preferably about 585 nm to about 595 nm, most preferred 590 nm. Preferably, such light is emitted by an LED device, preferably a handheld device, which, in certain preferred embodiments of the invention can emit non-coherent light.

In other preferred embodiments of the invention, the invention makes use of the light absorption of phenothiazine photosensitizers as outlined herein in the near infrared region. In certain embodiments of this type, the photosensitizer-activating light is in the near infrared region of the electromagnetic spectrum, preferably from about 780 nm to about 820 nm, more preferably around or at 810 nm such as from about 800 nm to about 820 nm, most preferred 810 nm. Near infrared light has the advantage that it is absorbed to a lesser extend by body components so that it can reach into deeper into the irradiated regions as defined above. The fact of the wavelength dependency of light scattering which is higher for longer wavelengths further contributes to this effect, and at wavelengths of at or around 810 nm the scattering coefficient is higher than the absorption coefficient, and furthermore the scattering is a forward scattering further contributing to the higher depth of penetration. Such light preferably has a depth of penetration of about 5 to 10 cm which makes it particularly suitable for irradiation from the outside of the body.

In the context of irradiation in the near infrared region of the electromagnetic spectrum, preferably from about 780 nm to about 820 nm, more preferably around or at 810 nm such as from about 800 nm to about 820 nm, most preferred about 810 nm, the present invention also provides a photodynamic treatment sexually transmissible infections, particularly for treatment of bacterial or viral infections of the urogenital tract or of the oropharynx or of the skin, in particular in the facial and/or genital area, by application of a photosensitizer such as a phenothiazine photosensitizer according to the definition as outlined above, more preferably selected from methylene blue, new methylene blue and toluidine blue, to a desired region, especially to a part of the subject's body in need thereof (such as those as described above) to be treated, preferably a human, and transcutaneous irradiation of said region.

Depending on the particular photosensitizer, it may be desired for efficient energy transfer to select a wavelength or wavelength region, respectively, at or at least near the absorption maximum of the photosensitizer. "Near the absorption maximum" in the context of the invention typically means a range around the absorption maximum of from about - 50 nm to about + 50 nm, preferably from about -20 nm to about + 20 nm, more preferably from about -10 nm to about + 10 nm of the absorption maximum. The absorption characteristics, including an absorption maximum of the photosensitizer, preferably of a phenothiazine derivative as described herein, is typically in the visible to near infrared range of the light spectrum, will depend on the biological setting, i.e. in which the photosensitizer is present as well as whether the subject is irradiated from outside of the body or within the body, and will also depend on and thus can be tuned by the components of a composition in which the photosensitizer(s) is present for application.

In other embodiments of the invention, the wavelength used for activating the photosensitizer is selected from regions of the, typically visible, absorption spectrum being more remote from an absorption maximum of the respective photosensitizer. In this context of the invention, the term "remote from an absorption maximum" of a photosensitizer for use in the invention refers to a wavelength.

The duration and number of times of irradiation of the part of the subject's body to be treated according to the invention varies according the specific photosensitizer, the wavelength or wavelength region of the light and on whether irradiation of the part of the subject's body to be treated is carried from the outside or inside of the body of the subject to be treated. Typically, the irradiation is carried out one or more times, such as 1, 2, 3, 4, 5, 6, 7, 8, 9, 10 times or more in one or more sessions at a day of treatment. The treatment can be repeated for two or more days, wherein the days of treatment can be uninterrupted or interrupted by one or more days where no treatment takes place. Preferably, the duration of the irradiation per irradiation incidence (i.e. one or more times as outlined above) is from about 1 min to about 20 min, more preferably from about 2 min to about 20 min, particularly preferred from about 2 min to about 8 min such as preferably about 2 min to about 3 min.

In preferred embodiments of the invention the part of the subject's body to be treated is irradiated, e.g. when irradiation is carried out from outside of the subject, with an energy density of about 50 to about 150 J/cm², preferably about 80 to about 120 J/cm,' more preferably about 90 to about 110 J/cm, most preferably about 100 J/cm². Preferred emitted light intensities, especially in the case of irradiation with non-coherent light, preferably by LED devices, more preferably handheld LED devices, such as broadband LED devices or LED devices emitting light at about or around about 590 nm (as outlined above) range from about 6000 Lux to about 120000 Lux, preferably from about 10000 Lux to about 110000 Lux, most preferred from about 40000 Lux to about 60000 Lux, such as, particularly preferred about 50000 Lux or about 100000 Lux.

In general, the treatment of the invention is directed against any infections agent causing a sexually transmissible infection. Preferred infective agents targeted by the inventive treatment are bacteria such as *Treponema pallidum,* in particular, subspecies pallidum (the infection causing Syphillis), *Neisseria gonorrhoeae* (causing Gonorrhea), *Chlamydia trachomatis,* and *Shigella* bacteria (causing Shigellosis), in particular *Shigella dyenteriae, Shigella flexneri, Shigella boydii* and/or *Shigella sonnei.* The inventive treatment is also intended to for treatment of protozoan infections such as infections by *Trichomonas vaginalis* (causing Trichomoniasis) and parasitic infections such as infections by *Sarcoptes scabiei,* preferably var. hominis (causing Scabies). Further, and particularly preferred, infective agents targeted by the inventive treatment are viruses, such as Human Papilloma Viruses (HPV), in particular the so-called high risk species (serotypes) (for causing carcinoma, see also below) such as preferably HPV 16, HPV 18, HPV 31, HPV 33, HPV 35, HPV 39, HPV 45, HPV 51, HPV 52, HPV 56, HPV 58, HPV 59, HPV 68, HPV 73 and/or HPV 82, but also the potentially high risk variants (serotypes) HPV 26, HPV 53, and/or HPV 66, Herpes viruses, preferably Herpes Simplex Virus, Hepatitis B Virus, Hepatitis C Virus and HIV (causing AIDS). As regards HPV infections the invention is also directed to HPV infections by so-called low risk HPV typically causing condylomae, specifically acuminate condylomae, including serotypes HPV 6 and/or HPV 11. Further sexually transmissible infections treatable by the regimens of the present invention include fungal infections, e.g. infections by *Candida albicans* such as infections by *Candida albicans* of the vagina or of the penis.

A particularly preferred application of the invention is the treatment and/or prevention of HPV infections, both in female and male, respectively, subjects, more preferably humans. HPV infections can be particularly dangerous for the infected subject, since HPV infection, particular by one or more of the above-mentioned high-risk and/or potentially high risk can cause cancer ((HPV 16, HPV 18, HPV 31 and/or HPV 33 are especially frequently associated with HPV-caused carcinoma) such as cervix carcinoma and its pre-forms, in particular cervix intraepithelial neoplasia (CIN), vaginal carcinoma, penis carcinoma, rectal carcinoma, mouth carcinoma and/or pharynx carcinoma.

One especially preferred anti-HPV treatment according to the invention relates to the treatment of HPV infection of the female cervix, in particular for the prevention of cervix carcinoma including prevention and/or treatment of pre-forms of cervix carcinoma, in particular cervical intraepithelial neoplasia (CIN). In such treatment, the female subject is preferably tested for potentially malignant process in the cervix by the Papanicolaou test ("Pap test" or "Pap smear"). According to the invention a Pap test result of Pap IIIId and/or Pap IV would preferably indicate the inventive treatment, preferably by intra-vaginal administration of the photosensitizer (at least one of them as outlined herein), most preferably to the cervical mucosa, and intra-vaginal irradiation of the cervix, preferably of the cervical mucosa, and/or transcutenaous irradiation of the cervix, preferably as outlined above. Also with respect to carcinomas in the mouth and/or pharynx, corresponding Pap tests are preferably performed, followed by the inventive treatment of administration of the photosensitizer(s) to the mouth and/or pharynx, and then irradiation of the respective part or parts of the oropharynx by intra-oral and/or intra-pharyngeal and/or transcutaneous irradiation as described above.

A particularly preferred treatment according to the invention involves treatment of CIN, typically involving PAP IIId or IV result and confirmed HPV infection, application of about 1 µg to about 15 µg, preferably about 5 µg to about 10 µg, of one or more phenothiazine photosensitizers as defined above, typically in form of an aqueous suspension or solution thereof, on the cervical mucosa and irradiation of the mucosa, preferably intra-vaginally, particularly preferred by using a light source adapted for intra-vaginal use, typically equipped with fibre optical elements, e.g. a waveguide, as outlined above, for about 2 to about 3 min with about 80000 Lux to about 120000 Lux, preferably about 100000 Lux at the required wavelength as outlined before.

It is to be understood that the present invention is directed to the treatment and/or prevention of all and any of the conditions or diseases, respectively, caused by the sexually transmissible infection as described and disclosed herein.

The present invention is also directed to a medical kit comprising the pharmaceutical device as described and defined herein and a light source emitting light comprising or having a wavelength causing the photosensitizer to be transferred to an active state. Preferred embodiments of such light sources have been elaborated above.

The present invention is also directed to a method for prevention and/or treatment of a sexually transmissible infection such as preferably those as outlined herein before, comprising the steps of (i) administering at least one photosensitizer to at least a part of the part or region of the body of the subject, preferably a human, to be treated, and (ii) irradiating said at least part or region of the subject's body with light having a wavelength causing the photosensitizer to be transferred in to an activated state. Preferred embodiments of photosensitizers and compositions containing same, administration regimens, light sources and light emitting devices, wavelengths, duration and times of irradiation for use in the inventive treatment method have already described herein above.

Further preferred details and embodiments the invention, in particular treatment conditions, body parts to be treated as well as devices, photosensitizers, light sources etc. have been described herein-before and will further be exemplified in the examples.

The present invention is further illustrated by the following non-limiting example:

### EXAMPLE

The effect of the inventive treatment on HPV as an example for sexually transmissible viruses was carried out with SV40 viruses which are recognized by the Robert-Koch-Institute, Federal Institute for Infectious Diseases of Germany, as surrogate for HPV in the examination of virucidal agents (Robert-Koch-Institute (2018) Epidemiologisches Bulletin No. 27, 255-259).

### Materials

### Irradiation device

### Broadband LED device emitting light at 590 nm/20000 Lux)

The device contains a hand piece allowing irradiation without loss of performance in a distance up to 105 cm with a light cone of approximately 1 cm diameter. In some experiments, the device is fixed on a tripod in a distance of 30 cm to ensure the same conditions for all tests.

### Irradiation conditions

Power density: 0.3 Watt
Irradiation Intensity: 100 Joule/cm²
Amount of sensitizer/well of 48-well plate: 0.1 %
0.01 %
0.001 %
0.0001 %
Test temperature (irradiation): 20°C ± 2°C
Incubation temperature (titration): 37°C ± 1°C

### Photosensitizer

Photolase Blueviolett Sensitizer 810: Methylene blue stock solution containing 1.07 % (w/v) methylene blue.

### Cells and viruses

SV-40 susceptible cells (CV-1 or BSC-1) are obtained from ATCC, Rockville, MD, USA)

### Media and auxiliary reagents

Eagle's Minimum Essential Medium with Earle's BSS (EMEM, Biozym Scientific GmbH, catalogue no. 880121)
Fetal calf serum (FCS, Sigma-Aldrich, article no. F 7524)
Aqua bidest. (SG ultrapure water system, type Ultra Clear; serial no. 86996-1)
PBS (Invitrogen, article no. 18912-014)
Trypsin-EDTA solution
Penicillin/ streptomycin (Sigma-Aldrich, article no. P-0781)
Trypsin stock solution [2.5 mg/ml]

### Methods

To analyse the efficacy of the inventive photoactivated treatement for inactivation of SV40, cells are cultivated in 48-well plates and infected with the virus before irradiation treatment. The following general steps were carried out:
(1) (Sub)culturing of cells in 48-well plates
(2) 150 µl cell suspension + 500 µl medium (EMEM 10 % FKS)
(3) Infection of the cells with 200 µl of virus-medium-mixture
(4) Pre-treatment and irradiation of the SV40-infected cells

### Preparation of test virus suspension

Propagation of SV-40 susceptible cells and their infection with SV40 virus are essentially carried out as described in Tremblay et al. (2001) Methods in Molecular Biology Vol. 165, pages 1 to 7.

Briefly, cells are cultured as monolayers in EMEM supplemented with 10 % Fetal Calf Serum (FCS) and 200 U/ml penicillin and 200 microgram/ml at 37°C under 5 % CO₂.

SV40 virus stock solution is prepared as follows: cells are cultured as above to 80 % to 100 % confluence in culture dishes. Medium is removed and replaced with 2 ml EMEM/2 % FCS containing an appropriate number SV40 plaque forming units (PFUs) to obtain a multiplicity of infection (MOI) between 0.1 and 0.01. Cells are incubated for 2 h with rocking the plates every 15 min. MEM/2% FCS are added to a final volume of 10 ml per culture dish of 10 cm in diameter. Dishes are cultured for 4 days, after which medium is replaced by fresh medium. Immediately after development of signs of cytopathic effects to complete destruction of the cell monolayer dishes are frozen at -20°C overnight, then thawed at room temperature. The Freeze/thaw cycle is repeated 2 times for a total of three cycles. Cellular debris is removed by low speed centrifugation. After aliquotation of the supernatant, test virus suspension is stored at -80 °C.

The viral test suspension is used for infection of the SV40 susceptible cells for the following experiments.

### Preparation of SV40 susceptible cells for irradiation treatment

SV40 susceptible cells of a 175 cm² cell culture flask are detached enzymatically with Trypsin-EDTA solution and taken up in a total of 60 ml of EMEM medium with 10 % fetal calf serum. 150 µl each of this cell suspension is transferred into six wells of a 48-well plate with a final volume of 650 µl by adding 500 µl EMEM/10 % FCS.

48-well plates are selected, since the diameter of one well corresponds to the diameter of the laser cone (light cone laser: approx. 1 cm diameter; well of 48-well plate: 1.04 cm diameter), which ensures that the cells are treated evenly throughout the entire well during radiation.

After three days of cultivation at 37 °C and 5 % CO₂, cells are washed two times with EMEM without FCS (2 x 200 µl per well) and incubated for further 3 h at 37 °C and 5 % CO₂. For virus infection, medium is removed from the individual wells and replaced by 200 µl virus-medium-mixture (500 µl SV40 virus suspension are mixed with 30 ml EMEM without FCS/ with penicillin/streptomycin, and trypsin). After an incubation period of 20 to 24 hours SV40-infected cells are used for irradiation treatment.

### Preparation of photosensitizer

The methylene blue photosensitzier Photolase^{®} Blueviolett Sensitizer 810 is used in the following concentrations based on the content of 1.07 % (w/v) methylene blue in the undiluted sensitizer:
(a) 0.1 % (w/v) (end concentration in 200 µl /well)
(b) 0.01 % (w/v) (end concentration in 200 µl /well)
(c) 0.001 % (w/v) (end concentration in 200 µl /well)
(d) 0.0001% (w/v) (end concentration in 200 µl /well)

### Irradiation procedure

For photodynamic inactivation of the SV40 virus first the undiluted or appropriately diluted (see above) photosensitizer solution (18.69 µl per well) is added to the SV40-infected cells for 1 minute. Thereafter, irradiation with the above laser device is performed with an intensity of 100 joule/cm² for 5.46 min under constant power of 0.3 watt/cm² and artificial laboratory light.

After treatment, plates are stored at -80 °C, successively.

### Recovery of the residual virus and determination of infectivity

For recovery of residual virus from the infected and treated cells, plates are subjected to a rapid freeze/thawing procedure. This is followed by mixing of cell suspension in each well by pipetting up and down 10 times to re-suspend the virus. Then, series of ten-fold dilutions of the suspensions are prepared in ice-cold maintenance medium, respectively. Finally, 100 µl of each dilution are placed in eight wells of a sterile polystyrene flat-bottomed plate with a preformed cell monolayer. Before the addition of the respective dilution, cells are washed twice with EMEM, and incubated for 3 hours in 100 µl EMEM supplemented with trypsin. The cells are incubated at 37 °C in a CO₂-atmosphere (5.0 % CO₂ content). After six days of incubation, cultures are observed for cytopathic effects. The infectious dose (TCID50) is calculated according to the method of Spearman (1908) Brit. J. Psychol. 2, 227-242, and Karber (1931) Arch. Exp. Path. Pharmak.; 162, 480-487.

### Controls

### (i) Virus control (VC)

Virus recovery is performed from non-treated infected cells as described under 3.5. The mean virus titre is used as reference for calculation of the reduction factor.

### (ii) Irradiation without photosensitizer

In addition, virus recovery is performed from infected and radiated cells without addition of the Photolase sensitizer.

### (iii) Treatment with photosensitizer (without irradiation)

Another virus recovery was performed from infected SV40-cells, treated with the Photolase sensitizer for 10 min under artificial laboratory light only (without radiation) as described in 3.5, whereby one Plate is wrapped up with aluminium foil immediately after addition of the dye (without radiation or light exposing) and stored and frozen in the dark until titration of residual virus.

### (iv) Cell culture control

Furthermore, a cell control (only addition of medium) is incorporated.

### Calculation of effectiveness

The virucidal effectiveness of the inventive treatment is evaluated by calculating the decrease in titre of the treated and radiated culture in comparison with the control titration of the culture without treatment (VC). The difference is given as reduction factor (RF).

Based on the EN 14476, the present photodynamic therapy system has a virucidal efficacy if the titre is reduced at least by 4 Iog10 steps after an irradiation treatment (EN 14476:2013+A2:2019: Chemical disinfectants and antiseptics - Quantitative suspension test for the evaluation of virucidal activity of chemicals disinfectants and antiseptics in human medicine test - Test method and requirements (phase 2, step 1)). This corresponds to an inactivation of ≥ 99.99 %

## Claims

1. A phenothiazine photosensitizer for use in the treatment of sexually transmissible infections.

2. The photosensitizer for use of claim 1 wherein the photosensitizer is topically applied to the urogenital tract or part thereof.

3. The photosensitizer for use of claim 2 wherein the photosensitizer applied by intra-vaginal and/or intra-urethral and/or intra-uterine administration.

4. The photosensitizer for use according to any one of the preceding claims wherein the photosensitizer is topically applied to the oropharynx or part thereof.

5. The photosensitizer for use of claim 4 wherein the photosensitizer applied by intra-nasal and/or intra-oral and/or intra-pharyngeal administration.

6. The photosensitizer for use according to any one of the preceding claims wherein the photosensitizer is topically applied to the skin.

7. The photosensitizer for use of claim 6 wherein the photosensitizer administered to the facial skin or parts thereof and/or the skin of the genital area or parts thereof and/or to peri-genital skin.

8. The photosensitizer for use according to any one of the preceding claims wherein the treatment comprises irradiating at least a part of the region of the body of a subject where the photosensitizer has been applied to with light comprising or having a wavelength causing the photosensitizer to be transferred to an active state.

9. The photosensitizer for use of claim 8 wherein the light is emitted by a light emitting diode (LED) device, preferably a handheld LED device.

10. The photosensitizer for use of claim 8 wherein the LED device is a laser LED device, preferably a handheld laser LED device, or a non-coherent light emitting LED device, preferably a broadband LED device.

11. The photosensitizer for use of claim 9 or 10 wherein the at least part of the body where the photosensitizer has been applied to is irradiated one or more times for a period of from 1 to 20 min, preferably from 2 to 8 min, more preferably from 2 to 3 min.

12. The photosensitizer for use of claim 11 wherein the at least part of the body where the photosensitizer has been applied to is irradiated from outside of the subject with an energy density of 50 to 150 J/cm², preferably 80 to 120 J/cm,' more preferably 90 to 110 J/cm, most preferably 100 J/cm².

13. The photosensitizer for use according to any one of the preceding claims wherein the phenothiazine photosensitizer is a photoactivatable phenothiazine derivative, preferably a photoactivatable phenothiazine dye, more preferably a phenothiazine derivative having a structure of the following formula (I) 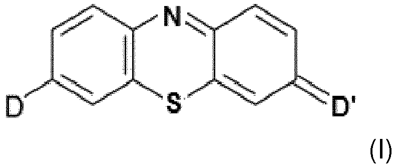 wherein
D and D' each are an electron donor group, and wherein the positions C-1, C-2, C-4, C-6, C-8 and/or C-9 of the phenothiazine ring system may be substituted by a substituent preferably selected from substituted or unsubstituted lower alkyl, preferably C₁₋₃-alkyl, particularly preferred methyl, or lower alkenyl, preferably C₂₋₃-alkenyl, amino, halo, and cyano. Preferably, D and D' are each independently a NRR' group with R and R' being preferably independently selected from H, substituted or unsubstituted lower alkyl, preferably C₁₋₃-alkyl, particularly preferred methyl, and lower alkenyl, preferably C₂₋₃-alkenyl.

14. The photosensitizer for use of claim 13 wherein the phenothiazine dye is selected from the group consisting of methylene blue, new methylene blue, toluidine blue and mixtures thereof.

15. The photosensitizer for use of claim 13 or 14 wherein the photoactivatable phenothiazine derivative is applied as a composition containing from 0.00001 (v/v) to 0.1 % (v/v), preferably from 0.0001 % (v/v) to 0.001 % (v/v), of said photoactivatable phenothiazine derivative.

16. The photosensitizer for use according to any one of claim 13 to 15 wherein the treatment comprises irradiating the least part of the body where the photosensitizer of a subject with light comprising or having a wavelength selected from 500 nm to 820 nm of the electromagnetic spectrum.

17. The photosensitizer for use of claim 16 wherein the light has a wavelength in the range of from 550 nm to 630 nm, preferably 570 to 610 nm, more preferably 585 nm to 595 nm, most preferred the light has a wavelength of 590 nm.

18. The photosensitizer for use of claim 16 wherein the light has a wavelength in the range of from 780 nm to 820 nm, preferably from about 800 nm to about 820 nm, most preferred the light has a wavelength of 810 nm.

19. The photosensitizer for use according to any one of the preceding claims wherein the sexually transmissible infection is caused by a virus, a bacterium, a fungus, a protozoon or a parasite.

20. The photosensitizer for use of claim 19 wherein the virus is selected from the group consisting of Human Papilloma Viruses, in particular the so-called high risk species (for causing carcinoma, see also below) such as preferably HPV 16, HPV 18, HPV 31, HPV 33, HPV 35, HPV 39, HPV 45, HPV 51, HPV 52, HPV 56, HPV 58, HPV 59, HPV 68, HPV 73 and/or HPV 82, but also the potentially high risk variants HPV 26, HPV53, and/or HPV 66, Herpes viruses, preferably Herpes Simplex Virus, Hepatitis B Virus, Hepatitis C Virus and Human Immunodeficiency Virus.

21. The photosensitizer for use of claim 20 wherein the HPV is selected from the group consisting of HPV 16, HPV 18, HPV 31, HPV 33, HPV 35, HPV 39, HPV 45, HPV 51, HPV 52, HPV 56, HPV 58, HPV 59, HPV 68, HPV 73 and HPV 82.

22. The photosensitizer for use of claim 20 wherein the HPV is selected from the group consisting of HPV 26, HPV53 and HPV 66.

23. The photosensitizer for use of claim 20 wherein the HPV is selected from the group consisting of HPV 6 and HPV 11.

24. The photosensitizer for use of claim 19 wherein the bacterium is selected from the group consisting of *Treponema pallidum,* preferably subspecies pallidum , *Neisseria gonorrhoeae, Chlamydia trachomatis, Shigella dyenteriae, Shigella flexneri, Shigella boydii* and *Shigella sonnei.*

25. The photosensitizer for use of claim 19 wherein the fungus is *Candida albicans.*

26. The photosensitizer for use of claim 19 wherein the protozoon is *Trichomonas vaginalis.*

27. The photosensitizer for use of claim 19 wherein the parasite is *Sarcoptes scabiei,* preferably var. hominis.
